(19) Europäisches Patentamt
European Patent Office
Office européen des brevets

(11) **EP 3 978 079 A1**

(12) **EUROPEAN PATENT APPLICATION**
published in accordance with Art. 153(4) EPC

(43) Date of publication:
**06.04.2022 Bulletin 2022/14**

(21) Application number: **20813728.1**

(22) Date of filing: **29.05.2020**

(51) International Patent Classification (IPC):
**A61Q 1/00** *(2006.01)*    **D01F 6/00** *(2006.01)*
**D01F 6/38** *(2006.01)*    **D04H 1/728** *(2012.01)*
**D01D 5/04** *(2006.01)*    **A61K 8/31** *(2006.01)*
**A61K 8/34** *(2006.01)*    **A61K 8/81** *(2006.01)*
**A61K 8/85** *(2006.01)*    **A61K 8/89** *(2006.01)*

(52) Cooperative Patent Classification (CPC):
**A61K 8/31; A61K 8/34; A61K 8/81; A61K 8/85;
A61K 8/89; A61Q 1/00; D01D 5/04; D01F 6/00;
D01F 6/38; D04H 1/728**

(86) International application number:
**PCT/JP2020/021417**

(87) International publication number:
**WO 2020/241847 (03.12.2020 Gazette 2020/49)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **31.05.2019    JP 2019103336
31.05.2019    JP 2019103337**

(71) Applicant: **Kao Corporation
Chuo-ku
Tokyo 103-8210 (JP)**

(72) Inventors:
• **ISHIDA, Kaori
Haga-gun, Tochigi 321-3497 (JP)**
• **FUJIMOTO, Tatsuya
Tokyo 131-8501 (JP)**
• **KOBAYASHI, Hideo
Kawasaki-shi, Kanagawa 210-0862 (JP)**
• **HOSOKAWA, Junji
Haga-gun, Tochigi 321-3497 (JP)**
• **HIRONO, Shingo
Cincinnati, Ohio 45214 (US)**

(74) Representative: **Hoffmann Eitle
Patent- und Rechtsanwälte PartmbB
Arabellastraße 30
81925 München (DE)**

(54) **FILM FORMING COMPOSITION AND ULTRA-FINE SHORT FIBERS**

(57)    Provided is a composition containing fine fibers for forming a coating film. The composition has excellent spreadability and provides a coating film with excellent uniformity by application to a skin.

A composition for forming a coating film comprising the following components (a) and (b):
(a) a fiber at 0.5 mass% or more and 10 mass% or less based on the total composition for forming a coating film. The fiber has an average fiber diameter of 0.1 $\mu$m or more and 7 $\mu$m or less and an aspect ratio [(average fiber length)/(average fiber diameter)] of 8 or more and 300 or less; and
(b) a volatile component at 15 mass% or more and 90 mass% or less based on the total composition for forming a coating film.

The [(average fiber diameter)$^2$/(fiber content)] ($\mu$m$^2$/mass%) of the composition is 0.005 or more and 7 or less, and the total content of the component (a) and the component (b) is 97 mass% or less.

**Description**

Field of the Invention

The present invention relates to a composition for forming a coating film capable of forming a film coating containing fine fibers on a skin surface.

The present invention also relates to an ultrafine short fiber for forming a thin film coating useful in cosmetics, external preparations for skin, coating agents, and the like.

Background of the Invention

Techniques for incorporating fibers into cosmetics are well known and widely used in mascara and the like. To make up a keratinous substance such as skin, a composition containing a fiber and a copolymer including a carboxylate group and a polydimethylsiloxane group in a physiologically acceptable medium (Patent Literature 1) has been reported. In addition, to reduce the irritation from cosmetics containing irritating components, a technique of incorporating fibers into cosmetics has also been reported. Further, to improve cosmetic durability, a skin cosmetic (Patent Literature 3) in which short fibers having a length of 0.1 to 5 mm are formulated has also been reported.

It has been also studied that an ultrafine short fiber is used as a filler or the like in a thin film in a cosmetic field, a pharmaceutical field, an electronic material field, or the like. For example, there has been reported a fiber dispersion (Patent Literature 4) in which the diameter of a single fiber according to the number average is 1 to 500 nm and the sum Pa of the single fiber ratios is 60% or more, an ultrafine short fiber (Patent Literature 5) in which the average fiber diameter is 1 000 nm or less and the average fiber length is 20 $\mu$m or less and the CV value of the fiber length is 55% or less, a method for producing a short fiber having an average diameter of 50 nm to 10 $\mu$m and an average length of 1 to 50 $\mu$m by cutting using a blade located at an interface or an intermediate phase of two or more liquid phases (Patent Literature 6), and a short fiber (Patent Literature 7) in which the coefficient of variation of the fiber diameter and the fiber length are 0 to 15 and 0 to 20, respectively.

(Patent Literature 1) JP-A-2002-193746
(Patent Literature 2) JP-A-2002-293718
(Patent Literature 3) JP-A-hei 7-196440

(Patent Literature 4) JP-A-2005-320506
(Patent Literature 5) JP-A-2009-114560
(Patent Literature 6) JP-A-2012-52271
(Patent Literature 7) JP-A-2007-92235

DISCLOSURE OF THE INVENTION

The present invention relates to a composition for forming a coating film. The composition comprises the following components (a) and (b).

(a) A fiber at 0.5 mass% or more and 10 mass% or less based on the composition for forming a coating film. The fiber has an average fiber diameter of 0.1 $\mu$m or more and 7 $\mu$m or less and an aspect ratio [(average fiber length)/(average fiber diameter)] of 8 or more and 300 or less; and
(b) A volatile component at 15 mass% or more and 90 mass% or less based on the total composition for forming a coating film.

In the present invention, [(average fiber diameter)$^2$/(fiber content)] ($\mu$m$^2$/mass%) is preferably 0.005 or more and 7 or less.

In the present invention, a total content of the component (a) and the component (b) is preferably 97 mass% or less.

The present invention relates to a method for producing a coating film on a surface of a skin. The method comprises applying the above composition for forming a coating film to the skin.

The present invention relates to a short fiber for forming a thin film. The short fiber has an average fiber diameter of 0.1 $\mu$m or more and 7 $\mu$m or less.

In the present invention, the short fiber preferably has an average fiber length of 20 μm or more and 300 μm or less.

In the present invention, a CV value of the fiber length is preferably 40% or more and 100% or less.

In the present invention, a proportion of the number of fibers having a fiber length of 40 μm or more in the whole fibers is preferably 5% or more.

The present invention also relates to a method for producing the above short fiber. The method comprises shortening nanofibers produced by an electrospinning method.

Brief Description of Drawings

Figure 1 is a schematic view illustrating the configuration of the electrostatic spraying device used for forming the fibers of the component (a)

Figure 2 shows a reference example of a SEM image with network formation.

Figure 3 shows a reference example of a SEM image without network formation.

Figure 4 shows another reference example of a SEM image with network formation.

Figure 5 shows another reference example of a SEM image without network formation.

Detailed Description of the Invention

The fiber diameter used in Patent Literatures 1 and 2 is as large as 0.9 dtex (= 10.7 μm), and the content of fibers is low, so that a fiber network is not formed, and there is an issue in the spreadability of a composition and the uniformity of a cosmetic film. In the skin cosmetic of Patent Literature 3, the content of fibers is low, so that the fiber network is not formed, and there is an issue in uniformity of the resulting cosmetic film.

Accordingly, the present invention provides a composition, containing fine fibers for forming a coating film, having excellent spreadability and providing a coating film with excellent uniformity, by application of the composition to a skin.

The short fibers of Patent Literatures 4 to 7 primarily aim at selectively producing fibers having a narrower fiber length distribution, and when the fibers are dispersed in a thin film, the strength and the like of the resulting thin film are improved, but the short fibers hardly form a network structure in the thin film, so that there is an issue that sufficient adhesivity may not be imparted to the thin film.

Accordingly, the present invention relates to provision of a short fiber for forming a thin film, which can impart adhesivity to the obtained thin film by forming a dense network structure when dispersed in the thin film.

Use of the composition for forming a coating film of the present invention facilitates to form a coating film excellent in adhesivity and durability.

Use of the short fiber of the present invention enables the short fibers to form the network structure in the resulting thin film, thereby imparting excellent adhesivity to the thin film.

The composition for forming a coating film of the present invention comprises the following components (a) and (b):

(a) a fiber having an average fiber diameter of 0.1 μm or more and 7 μm or less and an aspect ratio [(average fiber length)/(average fiber diameter)] of 8 or more and 300 or less; and
(b) a volatile component.

The component (a) is a fiber having an average fiber diameter of 0.1 μm or more and 7 μm or less and an aspect ratio [(average fiber length)/(average fiber diameter)] of 8 or more and 300 or less. The component (a) forms the network in a formed coating film to impart uniformity and adhesivity to the film. It can be determined by a scanning electron microscope (SEM) whether the fiber forms the network in the film. The network is a state in which fibers dispersed in the coating film have intersections with one another to provide gaps, where components contained in the composition for forming a coating film can be held in the gaps.

The fiber diameter is the diameter of a cross-section of a fiber in principle. Here, when the cross-section of the fiber is circular, the fiber diameter is the diameter of the circle, and when the cross-section of the fiber is elliptic, the fiber diameter is the major axis. The average fiber diameter of the fiber for use in the present invention is 0.1 μm or more and 7 μm or less from the viewpoint of the uniformity of the formed coating film.

In view of the practical formulation amount, the average fiber diameter is preferably 0.2 μm or more, more preferably 0.3 μm or more.

From the viewpoint of enhancing the capillary force of the fiber to improve adhesivity, the average fiber diameter

is preferably 5 μm or less, more preferably 4 μm or less, even more preferably 3 μm or less.

The fiber diameter can be measured by observing fibers at a magnification of 2 000 times or 5 000 times by SEM, randomly selecting 100 fibers with the exclusion of defects (e.g., lumps of fibers and intersection portions of fibers) among the two-dimensional images of the observed fibers, drawing a line orthogonal to the longer direction of the fiber, and directly reading the fiber diameter. An arithmetic average of these measured values is determined, and defined as the average fiber diameter. Since the fibers are dispersed in the composition for forming a coating film, the composition for forming a coating film is thinly applied to a substrate, and measurement is performed by SEM observation.

The fiber length is preferably 20 μm or more and 300 μm or less in terms of average fiber length from the view point of uniformity of the formed coating film and adhesivity of the resulting thin film.

From the viewpoint of easily forming the network, the fiber length is more preferably 25 μm or more, further more preferably 30 μm or more, even more preferably 40 μm or more.

From the viewpoint of suppressing entanglement and twisting of fibers during application of the composition, the fiber length is more preferably 250 μm or less, even more preferably 200 μm or less, especially preferably 150 μm or less.

The fiber length can be measured by observing fibers at a magnification of from 250 times to 750 times by SEM, randomly selecting 100 fibers with the exclusion of defects (e.g. lumps of fibers and intersection portions of fibers) among the two-dimensional images of the observed fibers, drawing a line orthogonal to the longer direction of the fiber, and directly reading the fiber length. An arithmetic average of these measured values is determined, and defined as the average fiber length.

The aspect ratio [(average fiber length)/(average fiber diameter)] is 8 or more and 300 or less from the viewpoint of the uniformity of the formed coating film and the durability of the film from formation of a uniform network.

The aspect ratio is preferably 10 or more, more preferably 15 or more, even more preferably 20 or more, especially preferably 25 or more, from the viewpoint of the uniformity of the formed coating film and the durability of the coating film from formation of a uniform network, and is especially preferably 40 or more from the viewpoint of increasing the entanglement of fibers to improve the strength of the thin film.

The aspect ratio is more preferably 250 or less, even more preferably 200 or less, from the viewpoint of the uniformity of the formed coating film and the durability of the coating film from formation of a uniform network.

The aspect ratio as used herein is not a value for one fiber, but a value calculated from the average fiber diameter determined in accordance with the method for measuring the fiber diameter and the average fiber length determined in accordance with the method for measuring the fiber length.

The CV value (coefficient of variation) of the fiber length of the (a) fiber is preferably 40% or more and 100% or less from the viewpoint of the uniformity of the formed coating film and formation of the network by the fiber in the film.

From the viewpoint of easily forming the network, the CV value is more preferably 42% or more, even more preferably 45% or more.

From the viewpoint of improving the storage stability of the composition, the CV value is more preferably 95% or less, even more preferably 90% or less, especially preferably 85% or less.

The CV value is a value which can be determined from (standard deviation of measured fiber lengths)/(average fiber length) × 100 [%]. The CV value is calculated from the measured values obtained during the measurement of the average fiber diameter and the average fiber length.

In the (a) fiber, the proportion of the number of fiber having a fiber length of 40 μm or more in the whole fiber is preferably 5% or more from the viewpoint of formation of a strong network in the film and the uniformity of the resulting coating film.

The fibers having a fiber length of 40 μm or more are contained more preferably at 8% or more, even more preferably at 15% or more from the viewpoint of increasing entanglement of the fibers to improve the strength of the thin film.

The upper limit of the length of the fiber having a fiber length of 40 μm or more is preferably 500 μm or less.

The fibers having a fiber length of 40 μm or more are contained preferably at 100% or less from the viewpoint of easily forming the network.

The (a) fiber is preferably a fiber in which the average fiber diameter is 0.1 μm or more and 7 μm or less, the average fiber length is 20 μm or more and 300 μm or less, the CV value of the fiber length is 40% or more and 100% or less, and the proportion of the number of fiber having a fiber length of 40 μm or more in whole fiber is 5% or more are more preferable as thin film forming short fibers.

The preferred ranges of the average fiber diameter, the average fiber length, the CV value of the fiber length and the proportion of the number of fibers having a fiber length of 40 μm or more are the same as the preferred ranges for the (a) fiber.

The short fiber of the present invention can be produced by including a step of shortening fibers obtained from a fiber-forming polymer by any of various spinning techniques. Here, the fiber-forming polymer is normally a thermoplastic or solvent-soluble chain polymer. A thermoplastic resin is preferable, and a thermoplastic resin having a weight average molecular weight of from $1.0 \times 10^4$ g/mol to $2.0 \times 10^5$ g/mol is more preferable. Use of water-insoluble polymers, among the fiber-forming polymers, is preferable from the viewpoint of maintaining the shape of fibers in the thin composition for forming a coating film.

The spinning method is preferably an electrospinning method (electrolytic spinning method) from the viewpoint of obtaining fibers having a small fiber diameter. For example, when the weight average molecular weight of biodegradable polyester is measured by gel permeation chromatography, the weight average molecular weight can be measured as a weight average molecular weight in terms of polystyrene under the following conditions. As polystyrene standard samples, polystyrene samples having known weight average molecular weighs and having different weight average molecular weights (e.g. monodisperse polystyrene manufactured by TOSOH CORPORATION (models: F450, F288, F128, F80, F40, F20, F10, F4, F1, A5000, A2500, A1000, A500 and A300) can be used to prepare a molecular weight calibration curve in advance, followed by comparing the calibration curve with the results of the measurement sample to perform measurement.

<Gel Permeation Chromatography Conditions>

- Measuring apparatus: HLC-8220GPC (manufactured by TOSOH CORPORATION)
- Column: GMHHR-H+GMHHR-H (manufactured by TOSOH CORPORATION)
- Eluent: 1 mmol FARMIN DM20 (Kao Corporation)/CHC13
- Eluent flow rate: 1.0 mL/min
- Column temperature: 40°C
- Detector: RI
- Sample concentration: 0.1 vol% (chloroform solution)
- Sample injection amount: 100 mL

The fiber of a water-insoluble polymer refers to one having a property such that in an environment at 1 atm and 23°C, weighed 1 g of the fiber immersed in 10 g of deionized water, and more than 0.5 g of the immersed polymer is undissolved after elapse of 24 hours.

Examples of the water-insoluble polymer include fully saponified polyvinyl alcohols which can be subjected to insoluble treatment after film formation, partially saponified polyvinyl alcohols which can be subjected to crosslinking treatment after film formation when used in combination with a crosslinker, oxazoline-modified silicones such as poly(N-propanoylethyleneimine)graft-dimethylsiloxane/y-aminopropylmethylsiloxane copolymers, polyvinyl acetal diethylaminoacetate, Zein (main component of corn protein), polyester resins such as polylactic acid (PLA), polybutylene succinate, polyethylene terephthalate (PET) and polybutylene terephthalate, acrylic resins such as polyacrylonitrile resins and polymethacrylic acid resins, polystyrene resins, polyvinyl butyral resins, polyvinyl acetal resins, polyurethane resins, polyamide resins, polyimide resins, polyamideimide resins, polypropylene resins, polyethylene resins, and various polypeptides (e.g. collagen, gelatin, fibrin and casein). These water-insoluble polymers can be used alone, or in combination of two or more thereof.

Among these water-insoluble polymers, one or more selected from the group consisting of fully saponified polyvinyl alcohols which can be subjected to insoluble treatment after film formation, partially saponified polyvinyl alcohols which can be subjected to crosslinking treatment after film formation when used in combination with a crosslinker, acrylic resins such as polymethacrylic acid resins, polyvinyl butyral resins, polyurethane resins, polylactic acid (PLA), oxazoline-modified silicones such as poly(N-propanoylethyleneimine)graft-dimethylsiloxane/y-aminopropylmethylsiloxane copolymers, polyvinyl acetal diethylaminoacetate and Zein can be used.

Among these, one or more selected from the group consisting of polyvinyl butyral resins, acrylic resins, polypropylene resins, polyesters such as polylactic acid, and polyurethane resins are more preferable from the viewpoint of ease of formation of nanofibers.

The acrylic resin is preferably an (octylacrylamide/hydroxypropyl acrylate/butylaminoethyl methacrylate) copolymer.

It is also preferable to use a biodegradable resin such as polylactic acid, polybutylene succinate, polyglycolic acid, polycaprolactone or polyhydroxyalkanoic acid from the viewpoint of reducing loads on the environment. The "biodegradability" herein means that the degree of biodegradation of polyester measured in accordance with JIS K6953-1 is 30% or more.

Examples of the fiber shortening treatment means include methods of cutting, shearing, fragmentation, pulverization, crushing or defibration, and for example, mechanical vortex pulverizers, impact pulverizers such as hammer pulverizers, jet pulverizers such as jet mills, media pulverizers such as ball mills and rod mills, cutter mill pulverizers, dry pulverizers such as disc mill pulverizers, media pulverizers using a liquid medium, wet pulverizers using a medialess pulverizer, and combinations thereof can be used.

As more preferred means for shortening fibers, a fiber assembly in which nanofibers are interlaced, e.g. a nonwoven fabric, is produced, and the fiber assembly is cut to an appropriate size, followed by using a mechanical vortex pulverizer, a cutter mill pulverizer, a disc mill pulverizer, a wet high-speed shearing medialess pulverizer or a wet high-pressure shearing medialess pulverizer. The fiber assembly includes not only nonwoven fabrics but also fiber assemblies having a predetermined thickness, such as cotton-like materials.

The content of the component (a) in the composition of the present invention is 0.5 mass% or more and 10 mass% or less from the viewpoint of the uniformity and adhesivity of the formed coating film, and ease of formation of the fiber network.

In view of the practical formulation amount in the composition, the content is preferably 0.7 mass% or more, more preferably 1 mass% or more.

In view of the realistic formulation amount in the composition, the content is preferably 8 mass% or less, more preferably 6 mass% or less.

For the content of the component (a) in the total composition for forming a coating film, first, of the fibers contained in the composition, fibers recognized as fibers of a water-insoluble polymer on the basis of the definition of a water-insoluble polymer are obtained. Subsequently, the fibers are washed with a solvent in which the fibers are insoluble, followed by filtering the fibers to obtain only fibers of the water-insoluble polymer. The solvent is preferably ethanol when the resin contained in the component (a) is an ester-based resin such as polylactic acid, and the solvent is preferably water when the resin is an acryl-based resin. The mass can be determined by measuring the fibers of the water-insoluble polymer, and the mass% of the fibers can be determined from a ratio to the weight of the composition before washing, i.e., (weight of component (a) after washing) / (weight of composition before washing).

The (b) volatile component exhibits an effect of improving the spreadability of the composition of the present invention and making the formed coating film even to improve adhesivity.

The volatility in the present invention means that the vapor pressure of a volatile component alone is 0.01 kPa or more and 106.66 kPa or less at 20°C.

Examples of the (b) volatile component include water, alcohol, amides, ketone, volatile silicone and volatile hydrocarbons, and from the viewpoint of ease of application, etc., one or more selected from the group consisting of water, alcohol, volatile silicone and volatile hydrocarbons are preferable.

Examples of the alcohol include a monohydric chain fatty acid alcohol, a monohydric cyclic fatty acid alcohol, or a monohydric aromatic alcohol is suitably used. Examples of the monohydric chain fatty acid alcohol include $C_1$-$C_6$ alcohols, examples of the monohydric alcohol include $C_4$-$C_6$ cyclic alcohols, and examples of the monohydric aromatic alcohol include benzyl alcohol and phenylethyl alcohol. Specific examples thereof include ethanol, isopropyl alcohol, butyl alcohol, phenylethyl alcohol, n-propanol and n-pentanol. One or more selected from the group consisting of these alcohols can be used.

Examples of the volatile silicone include dimethylpolysiloxane and cyclic silicone.

Examples of the volatile hydrocarbon include isododecane and hydrogenated polyisobutene.

The content of the (b) volatile component is 15 mass% or more and 90 mass% or less in the composition of the present invention from the viewpoint of the spreadability of the composition and the uniformity of the coating film.

From the viewpoint of use impression during or after application of the composition for forming a coating film to the skin or substrate surface, the content of the volatile component is more preferably 17 mass% or more, even more preferably 20 mass% or more, especially preferably 30 mass% or more.

From the viewpoint of the formability of the fiber network after application of the composition for forming a coating film to the skin or substrate surface, and the durability of the film, the content of the volatile component is preferably 87 mass% or less, more preferably 85 mass% or less.

The content of the volatile component can be measured by heating and/or decompressing a sample, and measuring a loss of the weight of the sample. The content of the volatile component can be measured from a loss of the weight of the sample when the sample is heated at 200°C using an infrared moisture gauge until the weight no longer changes.

In the composition of the present invention, the [(average fiber diameter)$^2$/ (fiber content)] ($\mu$m$^2$/mass%) is preferably in the range of 0.005 or more and 7 or less for ensuring that fibers form the network in the formed coating film, and the coating film has good uniformity and adhesivity. The fiber content means mass% of fibers in the composition for forming a coating film.

This value is preferably 0.02 or more, more preferably 0.03 or more, even more preferably 0.05 or more, from the viewpoint of the uniformity of the fiber network.

This value is preferably 6 or less, more preferably 5 or less, further more preferably 4 or less, even more preferably 3 or less, from the viewpoint of sufficiently forming the network of fibers in the film.

This value, i.e., [(average fiber diameter)$^2$/(fiber content)] ($\mu$m$^2$/mass%), is an index of the cumulative length of fibers contained in the composition, and the cumulative length decreases as this numerical value increases.

The total content of the component (a) and the component (b) in the composition of the present invention is 97 mass% or less from the viewpoint of the spreadability of the composition and the uniformity and adhesivity of the formed coating film.

In view of the practical formulation amount in the composition, the above-mentioned total content is preferably 92 mass% or less, more preferably 90 mass% or less, even more preferably 87 mass% or less.

In view of the practical formulation amount in the composition, the above-mentioned total content is preferably 16 mass% or more, more preferably 21 mass% or more, even more preferably 31 mass% or more.

The mass ratio of the component (a) to the component (b), (a/b), in the composition of the present invention is preferably 0.005 or more, more preferably 0.01 or more, from the viewpoint of the spreadability of the composition and the uniformity and adhesivity of the formed coating film.

In view of the practical formulation amount in the composition, the above-mentioned mass ratio is preferably 0.7 or less, more preferably 0.4 or less, even more preferably 0.25 or less, especially preferably 0.2 or less.

The composition of the present invention may contain (c) a nonvolatile oil agent, (d) a surfactant, (e) a polyol which is liquid at 20°C, a preservative and various powders, and a moisturizing agent other than the component (e), a water-soluble polymer, an amino acid, a pigment and the like, in addition to the components (a) and (b).

Examples of the (c) nonvolatile oil agent include an ester oil, a hydrocarbon oil, a higher alcohol, an ether oil, a silicone oil and a fluorine oil.

Of these, one or more selected from the group consisting of an ester oil, a hydrocarbon oil, an ether oil and a higher alcohol are more preferable from the viewpoint of imparting high durability to the film.

The content and the chemical structure of the component (c) can be identified by determining a molecular structure using a known technique such as an NMR (nuclear magnetic resonance apparatus), chromatography or IR analysis, or a combination thereof. By the above-described measurement, the content of the component (c) can be measured from, for example, the intensity of a measured value at a part showing the chemical structure.

As the ester oil, one or more selected from the group consisting of esters of a linear or branched fatty acid and a linear or branched alcohol or a polyhydric alcohol, and triglycerin fatty acid esters (triglyceride) can be used.

Specifically, one or more selected from the group consisting of isopropyl myristate, cetyl octanoate, octyldodecyl myristate, isopropyl palmitate, butyl stearate, hexyl laurate, myristyl myristate, decyl oleate, hexyldecyl dimethyloctanoate, cetyl lactate, myristyl lactate, lanolin acetate, isocetyl stearate, isocetyl isostearate, cholesteryl 12-hydroxystearate, ethylene glycol di(2-etylhexanoate), dipentaerythritol fatty acid ester, N-alkyl glycol monoisostearate, neopentyl glycol dicaprate, diisostearyl malate, glycerin di(2-heptylundecanoate), trimethylolpropane tri(2-ethylhexanoate), trimethylolpropane triisostearate, pentaerythritol tetra(2-ethylhexanoate), glyceryl tri(2-ethylhexanoate), trimethylolpropane triisostearate, cetyl 2-ethylhexanoate, 2-ethylhexylpalmitate, diethylhexyl naphthalenedicarboxylate, (C12-15) alkyl benzoate, cetearyl isononanoate, glycerin tri(caprylate/caprate), butylene glycol (dicaprylate/dicaprate), glyceryl trilaurate, glyceryl trimyristate, glyceryl tripalmitate, glyceryl triisostearate, glyceryl tri(2-heptylundecanoate), glyceryl tribehenate, tri-coconut oil fatty acid glyceryl, castor oil fatty acid methyl ester, oleyl oleate, 2-heptylundecyl palmitate, diisobutyl adipate, N-lauroyl-L-glutamic acid-2-octyldodecyl ester, di(2-heptylundecyl) adipate, ethyl laurate, di(2-ethylhexyl) sebacate, 2-hexyldecyl myristate, 2-hexyldecyl palmitate, 2-hexyldecyl adipate, diisopropyl sebacate, di(2-ethylhexyl)succinate, triethyl citrate, 2-ethylhexyl paramethoxycinnamate, tripropylene glycol dipivalate, polyglyceryl diisostearate and glyceryl behenate can be used.

Of these, at least one selected from the group consisting of octyldodecyl myristate, myristyl myristate, isocetyl stearate, isocetyl isostearate, cetearyl isononanoate, diisobutyl adipate, di(2-ethylhexyl)sebacate, isopropyl myristate, isopropyl palmitate, diisostearyl malate, neopentyl glycol dicaprate, (C12-15) alkyl benzoate and glycerin tri(caprylate/caprate) is preferable, at least one selected from the group consisting of isopropyl myristate, isopropyl palmitate, diisostearyl malate, neopentyl glycol dicaprate, (C12-15) alkyl benzoate and glycerin tri(caprylate/caprate) is more preferable, and one or more selected from the group consisting of neopentyl glycol dicaprate, C12-C15 alkyl benzoate, glycerin tri(caprylate/caprate), isopropyl myristate, polyglyceryl diisostearate and glyceryl behenate are even more preferable from the viewpoint of the adhesivity and the durability of the formed coating film.

The oil agent in the present invention has an HLB value of 10 or less, preferably 8 or less. The HLB value is an index of hydrophilic-lipophilic balance, and the present invention employs a value calculated by the following expression from Oda and Teramura et al.

$$\text{HLB} = (\Sigma \text{ inorganic value} / \Sigma \text{ organic value}) \times 10$$

Examples of the hydrocarbon oil include hydrocarbon oils which are liquid at 20°C, such as liquid paraffin, squalane, squalene, n-octane, n-heptane, cyclohexane, light isoparaffin and liquid isoparaffin; and hydrocarbon oils which are solid or semisolid at 20°C, such as vaseline, ceresin, paraffin wax, microcrystalline wax, ozokerite, hydrogenated polyisobutene, polyethylene wax and polyolefin wax. One or more selected from the group consisting of liquid paraffin, liquid isoparaffin, squalane and vaseline are preferable from the viewpoint of the durability of the formed coating film.

Examples of the ether oil include alkyl-1,3-dimethyl butyl ethers such as cetyl dimethyl butyl ether, ethylene glycol dioctyl ether, glycerol monooleyl ether and dicaprylyl ether, and one or more selected from the group consisting of these ether oils can be used.

Examples of the higher alcohol include higher alcohols having 12 to 20 carbon atoms. Specific examples thereof include cetyl alcohol, stearyl alcohol, isostearyl alcohol and oleyl alcohol, and one or more selected from the group consisting of these higher alcohols can be used. The higher alcohol is preferably cetyl alcohol.

Animal or vegetable oil including the ester oil and hydrocarbon oil can be used. Examples of the animal or vegetable oil include olive oil, jojoba oil, macadamia nut oil, meadowfoam oil, castor oil, safflower oil, sunflower oil, avocado oil, canola oil, apricot kernel oil, rice germ oil and rice bran oil. Olive oil is preferable as the animal or vegetable oil.

Examples of the silicone oil include dimethylpolysiloxane, polyether-modified silicone, amino-modified silicone, carboxy-modified silicone, methylphenylpolysiloxane, fatty acid-modified silicone, alcohol-modified silicone, fatty acid alcohol-modified silicone, epoxy-modified silicone, fluorine-modified silicone, cyclic silicone and alkyl-modified silicone. Dimethylpolysiloxane is preferable as the silicone oil.

Examples of the fluorine oil include perfluorodecalin, perfluoroadamantane, perfluorobutyltetrahydrofuran, perfluorooctane, perfluorononane, perfluoropentane, perfluorodecane, perfluorododecane and perfluoropolyether.

The content of the component (c) in the composition of the present invention is preferably 2 mass% or more, more preferably 5 mass% or more, even more preferably 8 mass% or more, from the viewpoint of the dispersibility of the component (a) and the adhesivity and durability of the formed coating film.

The content of the component (c) in the composition of the present invention is preferably 80 mass% or less, more preferably 75 mass% or less, even more preferably 65 mass% or less, from the viewpoint of the dispersibility of the component (a) and the adhesivity and durability of the formed coating film.

From the viewpoint of the adhesivity of the formed coating film, the content of the oil agent selected from the group consisting of a silicone oil and a fluorine oil is preferably 80 mass% or less (from 0 to 80 mass%), more preferably 75 mass% or less (from 0 to 75 mass%), even more preferably 70 mass% or less (from 0 to 70 mass%), based on the content of the component (c).

Examples of the (d) surfactant include nonionic surfactants, anionic surfactants and cationic surfactants, and examples thereof include polyoxyethylene-methylpolysiloxane copolymers, poly(oxyethylene-oxypropylene)methylpolysiloxane copolymers, crosslinked polyether-modified silicone, crosslinked alkyl polyether-modified silicone, cetyl dimethicone copolyol, propylene glycol monostearate, sorbitan monooleate, glyceryl stearate, polyoxyethylene hydrogenated castor oil, polyoxyethylene alkyl ethers, sorbitan sesquioleate and diglyceryl monooleate. One of these surfactants may be used, or two or more thereof may be used in combination.

The HLB value of the nonionic surfactant is preferably more than 10, more preferably 12 or more.

The surfactant is preferably nonionic surfactant, and anionic surfactant.

The nonionic surfactant is preferably polyoxyethylene sorbitan monostearate (20 E.O.) and polyoxyethylene sorbitan tristearate (20 E.O.).

The anionic surfactant is preferably N-stearoyl-L-glutamic acid.

Examples of the (e) polyol which is liquid at 20°C include alkylene glycols such as ethylene glycol, propylene glycol, 1,3-propanediol and 1,3-butanediol; polyalkylene glycols such as diethylene glycol, dipropylene glycol, polyethylene glycol having a weight average molecular weight of 2 000 g/mol or less, and polypropylene glycol; and glycerins such glycerin, diglycerin and triglycerin. Among these, ethylene glycol, propylene glycol, 1,3-butanediol, dipropylene glycol, polyethylene glycol having a weight average molecular weight of 2 000 g/mol or less, glycerin and diglycerin are preferable, propylene glycol, 1,3-butanediol and glycerin are more preferable, and propylene glycol and 1,3-butanediol are even more preferable.

Examples of the preservative include phenoxyethanol, methyl paraoxybenzoate, ethyl paraaminobenzoate, isobutyl paraoxybenzoate, isopropyl paraoxybenzoate, ethyl paraoxybenzoate, butyl paraoxybenzoate, propyl paraoxybenzoate, benzyl paraoxybenzoate and ethyl hexanediol.

The composition of the present invention can be produced by heating and mixing the above-described components as necessary in accordance with conventional methods.

In the composition of the present invention, the viscosity at 20°C is preferably 5 mPa·s or more, more preferably 10 mPa·s or more, even more preferably 10 000 mPa·s or more, from the viewpoint of ease of spreading.

In view of the realistic formulation amount, the viscosity at 20°C is preferably 50 000 mPa·s or less, more preferably 30 000 mPa·s or less, even more preferably 20 000 mPa·s or less.

The viscosity of the composition of the present invention can be measured with a Brookfield viscometer (VISCOMETER TV-10, TOKI SANGYO CO., LTD.) at 20°C, and the rotor and the rotation speed conform to the standard defined by the measuring device.

The composition of the present invention is for forming a coating film, and can form a uniform coating film on a surface of a skin or another substrate when applied to the skin or the substrate. In this coating film, volatile components are eliminated by volatilization to form a network of fibers. As a result, the resulting coating film exhibits excellent in uniformity and adhesivity.

When the composition of the present invention is applied to the skin, a film excellent in uniformity can be formed on the skin surface. Examples of the means for application of the composition to the skin include application with fingers, application with a spray, application using a tool such as a roller or a sponge, and application of a stick-shaped solid cosmetic. The viscosity at 20°C is preferably in the above-described range in terms of a range which enables application by hand.

According to the present invention, the coating film formed on the skin surface has good uniformity, excellent adhesivity and durability, and preferably good transparency.

Here, the thickness of the film depends on the amount of coating, and is preferably 0.3 $\mu$m or more and 30 $\mu$m or less, more preferably 0.5 $\mu$m or more and 20 $\mu$m or less, in normal use (amount of applied products per unit area: from 1 to 3 mg/cm$^2$). The thickness is measured with a contact thickness meter (LITEMATIC VL-50A manufactured by Mitutoyo Corporation) on the substrate after the application to the substrate. The substrate to be used here is made of PET.

Examples

Next, the present invention will be described in more detail by way of Examples.

[Production Example of Component (a)]

Example 1 is shown as a production example of short fibers.

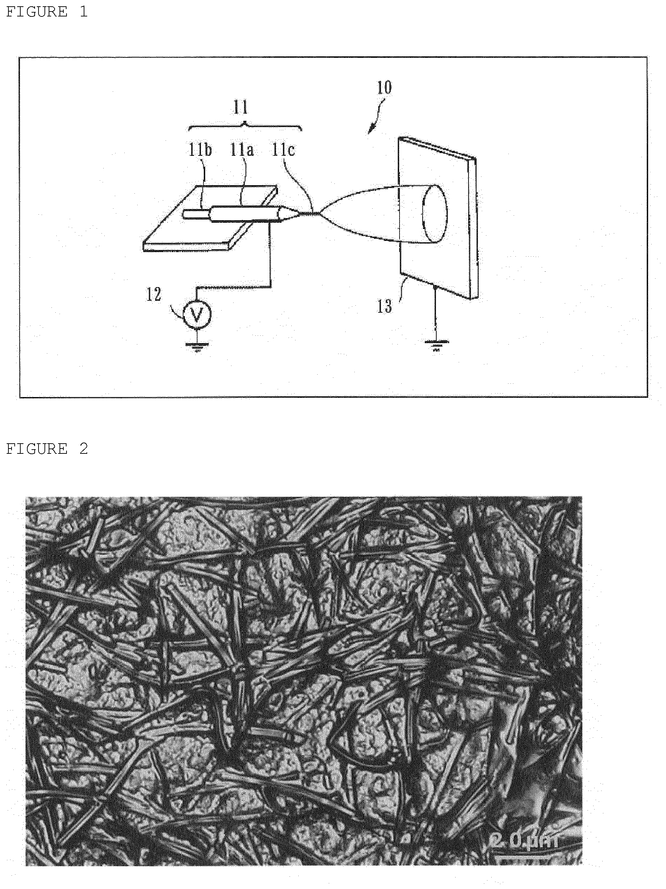

(1) The acrylic resin of Table 1, specifically an (octylacrylamide/hydroxypropyl acrylate/butylaminoethyl methacrylate) copolymer, was dissolved in ethanol to obtain a solution at 18 mass%. Using this solution, a nanofiber sheet was formed on a surface of a collector by an apparatus for an electrospinning method shown in Figure 1. Production conditions for the nanofibers are as follows.

- Applied voltage: 30 kV
- Distance between capillary and collector: 150 mm
- Discharged amount of aqueous solution: 12 mL/hour
- Environment: 25°C, 30%RH

(2) The obtained nanofiber sheet was cut to an appropriate size, a DISPER impeller was then attached to a stirring system (LABOLUTION (registered trademark) manufactured by PRIMIX Corporation), and shearing was performed at a rotation speed of 8,000 rpm for 15 minutes to obtain fibers.

In Examples 2 to 4, 7 to 11 and 13, fibers were produced in the same manner as in Example 1 except for the polymer concentrations, rotation speeds and shearing times shown in Table 1.

Example 5 is shown as a production example of short fibers.

The ester resins (PLA) of Table 1 were dissolved in chloroform and dimethylformamide (weight ratio 80 : 20) to obtain solutions at 20 mass%. With these solutions, nanofiber sheets were formed on a surface of a collector by the apparatus for the electrospinning method shown in Figure 1. Production conditions for the nanofibers are as follows.

- Applied voltage: 30 kV
- Distance between capillary and collector: 150 mm
- Discharged amount of aqueous solution: 12 mL/hour
- Environment: 25°C, 30%RH

(2) With a dispersing apparatus (MILDER manufactured by Pacific Machinery & Engineering Co. Ltd), the obtained nanofiber sheets were sheared by eight circulations through a circulation line at 13 500 rpm to obtain fibers.

In Examples 6 and 12, fibers were produced in the same manner as in Example 4 except for the polymer concentrations and the numbers of circulations in Table 1.

[Production Example of Compositions]

The obtained component (a) was added, and formulation was performed according to Table 1 to obtain compositions.

Table 1 shows the properties of the obtained fibers.

[Table 1]

| | Component | Example 1 | Example 2 | Example 3 | Example 4 | Example 5 | Example 6 | Example 7 | Example 8 | Example 9 |
|---|---|---|---|---|---|---|---|---|---|---|
| | | Acrylic resin (*1) | Acrylic resin (*1) | Acrylic resin (*1) | Acrylic resin (*1) | Ester resin (*2) | Ester resin (*2) | Acrylic resin (*1) | Acrylic resin (*1) | Acrylic resin (*1) |
| | Polymer concentration [%] | 18 | 18 | 24 | 24 | 20 | 20 | 18 | 18 | 27 |
| | Stirring method | DISPER | DISPER | DISPER | DISPER | MILDER | MILDER | DISPER | DISPER | DISPER |
| | Rotation speed [rpm] | 8000 | 8000 | 4000 | 4000 | 13500 | 13500 | 5000 | 5000 | 2500 |
| | Shearing time [min] (with DISPER) | 15 | 15 | 10 | 10 | - | - | 30 | 30 | 20 |
| | Number of circulations [times] (with MILDER) | - | - | - | - | 8 | 8 | - | - | - |
| (X) | Average fiber diameter [$\mu$m] | 0.5 | 0.5 | 2.0 | 2.0 | 0.7 | 0.7 | 0.5 | 0.5 | 4.0 |
| | [(average fiber diameter)$^2$/(fiber content)] [$\mu$m$^2$/mass%] | 0.063 | 0.063 | 1.000 | 1.000 | 0.123 | 0.123 | 0.063 | 0.063 | 4.000 |
| (Y) | Average fiber length [$\mu$m] | 30 | 30 | 40 | 40 | 45 | 45 | 50 | 50 | 100 |
| | Aspect ratio Y/X | 60 | 60 | 20 | 20 | 64 | 64 | 100 | 100 | 25 |
| (a) | Fiber content [mass%] | 4.00 | 4.00 | 4.00 | 4.00 | 4.00 | 4.00 | 4.00 | 4.00 | 4.00 |
| (c) | Neopentyl glycol dicaprate (*4) | 0.00 | 0.00 | 0.00 | 0.00 | 0.00 | 0.00 | 0.00 | 3.00 | 17.50 |
| | Polyglyceryl diisostearate (*5) | 0.00 | 0.00 | 0.00 | 0.00 | 0.00 | 0.00 | 0.00 | 1.00 | 0.00 |
| | Olive oil (*3) | 0.00 | 0.00 | 0.00 | 0.00 | 0.00 | 0.00 | 0.00 | 1.00 | 0.00 |
| | Glyceryl behenate (*6) | 0.00 | 0.00 | 0.00 | 0.00 | 0.00 | 0.00 | 0.00 | 1.00 | 0.00 |
| | Vaseline | 0.00 | 0.00 | 0.00 | 0.00 | 0.00 | 0.00 | 0.00 | 1.00 | 0.00 |
| | Squalane | 0.00 | 0.00 | 0.00 | 0.00 | 0.00 | 0.00 | 0.00 | 2.50 | 0.00 |
| | Cetyl alcohol | 0.00 | 0.00 | 0.00 | 0.00 | 0.00 | 0.00 | 0.00 | 1.00 | 0.00 |
| | Silicone oil 10CS(*7) | 0.00 | 0.00 | 0.00 | 0.00 | 0.00 | 0.00 | 0.00 | 1.00 | 0.00 |
| | Silicone oil 50CS(*8) | 0.00 | 0.00 | 0.00 | 0.00 | 0.00 | 0.00 | 0.00 | 5.00 | 0.00 |
| | Silicone oil KSG16(*9) | 0.00 | 0.00 | 0.00 | 0.00 | 0.00 | 0.00 | 0.00 | 0.50 | 0.00 |

EP 3 978 079 A1

(continued)

| | Component | Example 1 | Example 2 | Example 3 | Example 4 | Example 5 | Example 6 | Example 7 | Example 8 | Example 9 |
|---|---|---|---|---|---|---|---|---|---|---|
| | | Acrylic resin (*1) | Acrylic resin (*1) | Acrylic resin (*1) | Acrylic resin (*1) | Ester resin (*2) | Ester resin (*2) | Acrylic resin (*1) | Acrylic resin (*1) | Acrylic resin (*1) |
| (b) | Purified water | 84.00 | 19.00 | 84.00 | 19.00 | 68.87 | 3.87 | 84.00 | 53.50 | 76.30 |
| | Ethanol | 0.00 | 0.00 | 0.00 | 0.00 | 15.00 | 15.00 | 0.00 | 0.00 | 0.00 |
| (e) | Glycerin (86%) | 10.00 | 75.00 | 10.00 | 75.00 | 10.00 | 75.00 | 10.00 | 16.00 | 0.00 |
| | 1,3-butylene glycol | 0.00 | 0.00 | 0.00 | 0.00 | 0.00 | 0.00 | 0.00 | 5.00 | 0.00 |
| | Polyethylene glycol(*10) | 0.00 | 0.00 | 0.00 | 0.00 | 0.00 | 0.00 | 0.00 | 3.00 | 0.00 |
| (d) | Polyoxyethylene sorbitan monostearate (20E.O.) (*11) | 1.50 | 1.50 | 1.50 | 1.50 | 1.50 | 1.50 | 1.50 | 0.00 | 0.00 |
| | Polyoxyethylene sorbitan tristearate (20E.O.) (*12) | 0.00 | 0.00 | 0.00 | 0.00 | 0.00 | 0.00 | 0.00 | 0.00 | 1.60 |
| | N-stearoyl-L-glutamic acid (*13) | 0.00 | 0.00 | 0.00 | 0.00 | 0.00 | 0.00 | 0.00 | 0.60 | 0.00 |
| | Sodium acrylate-sodium acryloyldimethyltaurate copolymer (*14) | 0.50 | 0.50 | 0.50 | 0.50 | 0.30 | 0.30 | 0.50 | 0.00 | 0.00 |
| | Acrylic acid-alkyl methacrylate copolymer (*15) | 0.00 | 0.00 | 0.00 | 0.00 | 0.20 | 0.20 | 0.00 | 0.00 | 0.15 |
| | Carboxyvinyl polymer (*16) | 0.00 | 0.00 | 0.00 | 0.00 | 0.00 | 0.00 | 0.00 | 0.15 | 0.00 |
| | Xanthane gum | 0.00 | 0.00 | 0.00 | 0.00 | 0.00 | 0.00 | 0.00 | 0.10 | 0.00 |
| | L-arginine | 0.00 | 0.00 | 0.00 | 0.00 | 0.00 | 0.00 | 0.00 | 0.32 | 0.00 |
| | Calcium hydroxide (48%) | 0.00 | 0.00 | 0.00 | 0.00 | 0.13 | 0.13 | 0.00 | 0.23 | 0.10 |
| | Phenoxyethanol | 0.00 | 0.00 | 0.00 | 0.00 | 0.00 | 0.00 | 0.00 | 0.10 | 0.35 |
| | Total | 100.00 | 100.00 | 100.00 | 100.00 | 100.00 | 100.00 | 100.00 | 100.00 | 100.00 |
| | Amount of (b) volatile components | 84.00 | 19.00 | 84.00 | 19.00 | 83.87 | 18.87 | 84.00 | 53.50 | 76.30 |
| | (a)+(b) (a)/(b) | 88.00 | 23.00 | 88.00 | 23.00 | 87.87 | 22.87 | 88.00 | 57.50 | 80.30 |
| | | 0.048 | 0.211 | 0.048 | 0.211 | 0.048 | 0.212 | 0.048 | 0.075 | 0.052 |
| | Viscosity of composition [mPa·s] | 15000 | 15000 | 15000 | 15000 | 15000 | 15000 | 15000 | 30000 | 15000 |

(continued)

| Component | Example 1 | Example 2 | Example 3 | Example 4 | Example 5 | Example 6 | Example 7 | Example 8 | Example 9 |
|---|---|---|---|---|---|---|---|---|---|
| | Acrylic resin (*1) | Acrylic resin (*1) | Acrylic resin (*1) | Acrylic resin (*1) | Ester resin (*2) | Ester resin (*2) | Acrylic resin (*1) | Acrylic resin (*1) | Acrylic resin (*1) |
| CV value of fiber length [%] | 51 | 51 | 64 | 64 | 82 | 82 | 70 | 70 | 59 |
| Content of fibers having a fiber length of 40 $\mu$m or more in all fiber [%] | 25 | 25 | 66 | 66 | 82 | 82 | 56 | 56 | 59 |
| Average molecular weight of fibers [g/mol] | 80000 | 80000 | 80000 | 80000 | 15000 | 15000 | 80000 | 80000 | 80000 |
| (1) Adhesivity | 4 | 3 | 4 | 3 | 4 | 3 | 4 | 4 | 3 |
| (2) Uniformity | 4 | 4 | 4 | 4 | 4 | 4 | 4 | 4 | 4 |
| (3) Spreadability | 4 | 4 | 4 | 4 | 4 | 4 | 4 | 4 | 4 |
| (4) Network formation | formed | formed | formed | formed | formed | formed | formed | formed | formed |

*1: AMPHOMER 28-4910 (Akzo Nobel N.V.)
*2: Polylactic Acid Ingeo6252D (Natureworks)
*3: CROPURE OL-LQ (Croda Japan KK)
*4: ESTEMOL N-01 (The Nisshin Oillio Group, Ltd.)
*5: COSMOL 42V (The Nisshin Oillio Group, Ltd.)
*6: SUNSOFT No. 8100-C (Taiyo Kagaku Co., Ltd.)
*7: SILICONE KF-96A-10CS (Shin-Etsu Chemical Co., Ltd.)
*8: SILICONE KF-96A-50CS (Shin-Etsu Chemical Co., Ltd.)
*9: SILICONE KSG-16 (Shin-Etsu Chemical Co., Ltd.)
*10: PEG-1540 (-G) (NOF CORPORATION)
*11: RHEODOL TW-S120V (Kao Corporation)
*12: RHEODOL TW-S320V (Kao Corporation)
*13: AMISOFT HA-P (Ajinomoto Co., Inc.)
*14: SIMULGEL EG (SEPPIC)
*15: PEMULEN TR-1 (Lubrizol Advanced Materials)
*16: NYLON FIBER 0.5-6D (Cosmeterials KK)

Of these, *2 is biodegradable.

Examples 1 to 9

The composition for forming a coating film of Table 1 were produced, and each uniformly applied to an artificial leather to form a film. The properties of the formed coating films were evaluated.

(Evaluation Methods)

(1) Adhesivity

The composition for forming a coating film was applied to the artificial leather at 2 mg/cm$^2$, and transferability after drying for 15 minutes was evaluated. For the transferability, black paper was pressed against the fibers, followed by visually evaluating the transferability of the fibers on the basis of the following criteria.

4: Not transferred.
3: A slight but recognizable part is transferred (less than 40%).
2: Transferred at 40% or more and less than 70%.
1: Transferred at 70% or more.

(2) Uniformity

The composition for forming a coating film was applied to the artificial leather at 2 mg/cm$^2$ with a finger (10 to 20 gf), and the uniformity of the applied film was visually evaluated on the following criteria.

4: No irregularity is found.
3: Irregularity is visible at less than 30% of the total applied area.
2: Irregularity is visible at 30% or more and less than 70% of the total applied area.
1: Irregularity is present at 70% or more of the total applied area.

(3) Spreadability

To each of ten men and women in their twenties to thirties, the composition for forming a coating film was applied at 2 mg/cm$^2$ (applied site: arm), ease of spreading here was evaluated, and an average value was calculated and rounded off to the nearest integer. In spreading of the composition for forming a coating film, the composition was taken by a dominant hand, applied to the arm of the other hand, and spread over the arm to the extent that the formed coating film became transparent. A region of 5 cm × 5 cm was marked as a portion to which the composition was applied, and 50 mg of the composition was taken by the hand, and uniformly spread. In application of the composition, a site where the arm had no scar and little arm hair was selected. The criteria are as follows.

4: Very easily spread.
3: Easily spread.
2: Hardly spread (easily twisted).
1: Difficult to spread.

(4) Network formation

It was evaluated by SEM whether a network was formed.

In evaluation of network formation, when fibers each had two or more intersections with other fibers, and presence of gaps surrounded by the fibers was recognizable overall, it was determined that the network was formed.

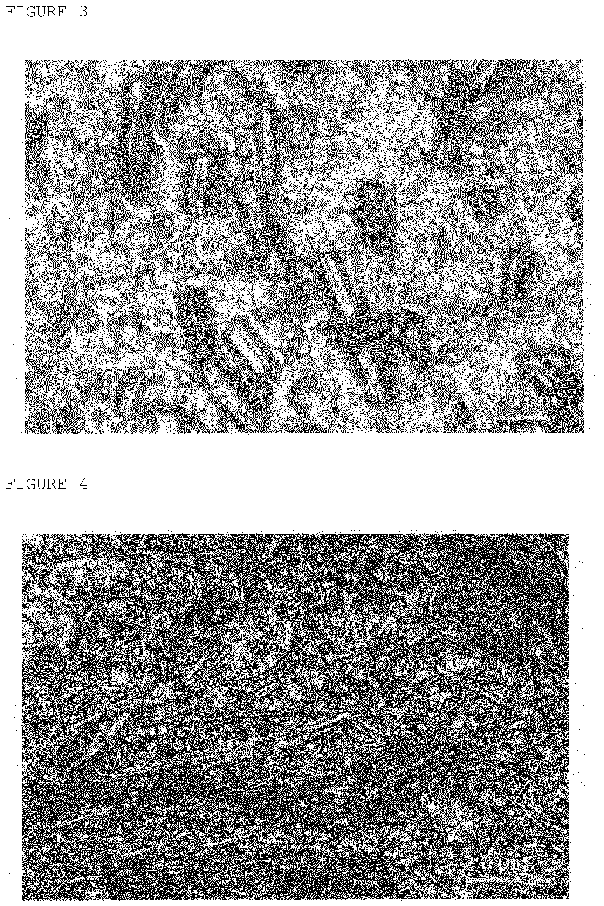

Table 1 shows the results. Figure 2 shows a SEM reference image in which the network is formed and Figure 3 shows a SEM image in which the network is not formed, where on the basis of the images, whether the network was formed or not was determined in Examples and Comparative Examples.

Examples 10 to 13 and Comparative Examples 1 and 2

The composition for forming a coating films of Table 2 were produced, and each uniformly applied to an artificial leather to form a film. The properties of the formed coating films were evaluated. The evaluation method is the same as in Examples 1 to 9.

[Table 2]

| | Component | Example 10 | Example 11 | Example 12 | Example 13 | Comparative Example 1 | Comparative Example 2 |
|---|---|---|---|---|---|---|---|
| | | Acrylic resin (*1) | Acrylic resin (*1) | Ester resin (*2) | Acrylic resin (*1) | Nylon (*16) | Acrylic resin (*1) |
| | Polymer concentration [%] | 18 | 18 | 20 | 18 | - | 18 |
| | Stirring method | DISPER | DISPER | MILDER | DISPER | - | DISPER |
| | Rotation speed [rpm] | 5000 | 5000 | 13500 | 3500 | | 1000 |
| | Shearing time [min] (with DISPER) | 30 | 30 | - | 15 | - | 90 |
| | Number of circulations times with MILDER) | - | | 8 | | | |
| (X) | Averaae fiber diameter [μm] | 0.5 | 0.5 | 0.7 | 0.5 | 4.3 | 0.5 |
| | [(averaae fiber diameter)$^2$/(fiber content) [μm$^2$/mass%] | 0.063 | 0.063 | 0.123 | 0.063 | 36.980 | 2.924 |
| (Y) | Average fiber length [μm] | 50 | 50 | 45 | 88 | 500 | 9 18 |
| (a) | Aspect ratio Y/X | 100 | 100 | 64 | 176 | 116 | 18 |
| | Fiber content [mass%] | 1.00 | 0.09 | 4.00 | 4.00 0.09 | 0.50 | 0.09 |
| (b) | Purified water | 72.00 | 67.00 | 0.09 | 0.09 | 42.00 | 62.91 |
| | Ethanol | 0.00 | 0.00 | 68.74 | 0.00 | 20.00 | 0.09 |
| (e) | Glycerin 86% | 25.00 | 0.09 | 10.00 | 0.09 | 0.09 10.00 | 0.09 10.00 |
| | 1,3-butylene glycol | 0.00 | 0.00 | 15.00 | 0.09 | 0.09 | 0.09 |
| | Polyethylene qlycol(*10) | 0.00 | 0.00 | 0.00 | 0.00 | 0.00 | 0.00 |

(continued)

| (d) | Component | Example 10 | Example 11 | Example 12 | Example 13 | Comparative Example 1 | Comparative Example 2 |
|---|---|---|---|---|---|---|---|
| | | Acrylic resin (*1) | Acrylic resin (*1) | Ester resin (*2) | Acrylic resin (*1) | Nylon (*16) | Acrylic resin (*1) |
| | Polyoxyethylene sorbitan monostearate (20E.O.)(*11) | 1.50 | 1.50 | 1.50 | 1.50 | 1.50 | 1.50 |
| | Polvoxyethylene sorbitan tristearate (20E.O.)(*12) | 0.00 | 0.00 | 0.00 | 0.00 | 0.00 | 0.00 |
| | N-stearoyl-L-glutamid acid (*13) | 0.00 | 0.00 | 0.00 | 0.00 | 0.00 | 0.00 |
| | Sodium acrylate-sodium acryloyldimethyltaurate copolymer (*14) | 0.50 0.09 | 0.50 0.09 | 0.00 0.09 | 0.50 0.09 | 0.00 | 0.00 |
| | Acrylic acid-alkyl methacrylate copolymer (*15) | 0.00 | 0.00 | 0.70 | 0.00 | 1.00 | 0.50 |
| | Aminomethyl propanol | 0.00 | 0.00 | 0.06 | 0.00 | 0.00 | 0.00 |
| | Total | 100.00 | 100.00 | 100.00 | 100.00 | 100.00 0.09 | 100.00 0.09 |
| | Amount of (b) volatile components | 72.00 | 67.00 | 68.74 | 0.09 | 62.00 | 0.09 63.00 |
| | (a)+(b) | 73.00 | 73.00 | 72.74 | 73.00 | 62.50 | 63.00 |
| | (a)/(b) | 0.014 | 0.090 | 0.058 | 0.058 | 0.008 | 0.001 |
| | Viscosity of composition [mPa·s] | 10000 | 25000 | 17000 | 12000 | 18000 | 22000 |
| | CV value of fiber length | 70 | 70 | 82 | 60 | - | 48 |
| | Content of fibers having a fiber length of 40 $\mu$m or more in all fiber [%] | 56 | 56 | 82 | 73 | 100 | 0.06 |
| | Average molecular weight of fibers [g/mol| | 80000 | 80000 | 15000 | 80000 | N.D. | 80000 |
| | (1) Adhesivity | 3 | 3 | 4 | 4 | 2 | 2 |
| | (2) Uniformity | 4 | 4 | 4 | 4 | 2 | 4 |
| | (3) Spreadability | 4 | 4 | 4 | 4 | 2 | 4 |
| | (4) Network formation | formed | formed | formed | formed | not formed | not formed |

[Production Example of Short Fibers]

Example 14 is shown as a production example of short fibers.

(1) The acrylic resin of Table 3, specifically an (octylacrylamide/hydroxypropyl acrylate/butylaminoethyl methacrylate) copolymer, was dissolved in ethanol to obtain a solution at 18 mass%. Using this solution, a nanofiber sheet was formed on a surface of a collector by an apparatus for an electrospinning method shown in Figure 1. Production conditions for the nanofibers are as follows.

- Applied voltage: 30 kV

- Capillary-collector distance: 150 mm
- Aqueous solution discharge amount: 12 mL/hour
- Environment: 25°C, 30%RH

(2) The obtained nanofiber sheet was cut to an appropriate size, a DISPER impeller was then attached to a stirring system (LABOLUTION (registered trademark) manufactured by PRIMIX Corporation), and shearing was performed at a rotation speed of 5 000 rpm for 25 minutes to obtain fibers.

In Examples 15, 16 and 18 to 21, fibers were produced in the same manner as in Example 14 except for the polymer concentrations, rotation speeds and shearing times shown in Table 3.

Example 17 is shown as a production example of short fibers.

The ester resins (PLA) of Table 1 were dissolved in chloroform and dimethylformamide (weight ratio 80 : 20) to obtain solutions at 20 mass%. Using these solutions, nanofiber sheets were formed on a surface of a collector by the apparatus for the electrospinning method shown in Figure 1. Production conditions for the nanofibers are as follows.

- Applied voltage: 30 kV
- Capillary-collector distance: 150 mm
- Aqueous solution discharge amount: 12 mL/hour
- Environment: 25°C, 30%RH

(2) Using a dispersing apparatus (MILDER manufactured by Pacific Machinery & Engineering Co. Ltd), the obtained nanofiber sheets were sheared by eight circulations through a circulation line at 13 500 rpm to obtain fibers.

In Examples 22 and 23, fibers were produced in the same manner as in Example 17 except for the polymer concentrations and the numbers of circulations in Table 3.

[Production Example of Compositions]

The obtained short fibers were formulated in accordance with the formulas of Table 4 to obtain thin composition for forming a coating films.

Tables 3 and 4 show the properties and formulas of the obtained fibers.

[Table 3]

| | Example | Example | Example | Example | Example | Example | Example | Example | Example | Example |
|---|---|---|---|---|---|---|---|---|---|---|
| | 14 | 15 | 16 | 17 | 18 | 19 | 20 | 21 | 22 | 23 |
| Resin raw material | Acrylic resin (*17) | Acrylic resin (*17) | Acrylic resin (*17) | Ester resin (*18) | Acrylic resin (*17) | Acrylic resin (*17) | Acrylic resin (*17) | Acrylic resin (*17) | Ester resin (*18) | Ester resin (*18) |
| Polymer concentration [%] | 18 | 18 | 27 | 20 | 18 | 18 | 24 | 18 | 23 | 19.5 |
| Stirring method | DISPER | DISPER | DISPER | MILDER | DISPER | DISPER | DISPER | DISPER | MILDER | MILDER |
| Rotation speed [rpm] | 5000 | 8000 | 2500 | 13500 | 5000 | 5000 | 3500 | 5000 | 13500 | 13500 |
| Shearing time [min] (with DISPER) | 25 | 15 | 15 | - | 20 | 20 | 20 | 15 | - | - |
| Number of circulations [times] (with MILDER) | - | - | - | 8 | - | - | - | - | 3 | 5 |
| Average fiber diameter [μm] | 0.5 | 0.5 | 4.0 | 0.7 | 0.5 | 0.5 | 2.0 | 0.5 | 1.5 | 0.6 |
| Average fiber length [μm] | 64.7 | 24.5 | 118.9 | 43.9 | 71.3 | 71.3 | 64.2 | 88.0 | 98.4 | 70.9 |
| Aspect ratio | 129.4 | 49.1 | 29.7 | 62.7 | 142.7 | 142.7 | 32.1 | 176.0 | 65.6 | 118.2 |
| CV value of fiber length[%] | 47.2 | 50.8 | 50.6 | 82.0 | 62.4 | 62.4 | 50.1 | 59.8 | 65.6 | 77.2 |
| Proportion of number of fibers having a fiber length of 40 μm or more [%] | 79.0 | 8.0 | 96.5 | 40.0 | 75.1 | 75.1 | 77.1 | 73.0 | 61.4 | 52.2 |
| Formulation amount of fibers [%] | 2.0 | 2.0 | 4.0 | 2.0 | 1.0 | 0.5 | 1.0 | 4.0 | 4.0 | 4.0 |
| (average fiber diameter)$^2$/ (formulation amount) | 0.125 | 0.125 | 4.000 | 0.245 | 0.250 | 0.500 | 4.000 | 0.063 | 0.563 | 0.090 |
| Network formability | formed | formed | formed | formed | formed | formed | formed | formed | formed | formed |
| Adhesivity (artificial leather) | 4 | 4 | 4 | 4 | 4 | 4 | 4 | 4 | 4 | 4 |
| Adhesivity (PET film) | 4 | 4 | 3 | 4 | 4 | 4 | 3 | 4 | 3 | 3 |
| Remark | Formula A | Formula A | Formula A | Formula B | Formula B | Formula B | Formula B | Formula A | Formula C | Formula C |

(*17)AMPHOMER 28-4910 (manufactured by Akzo Nobel N.V.) (octylacrylamide/hydroxypropyl acrylate/butylaminoethyl methacrylate) copolymer (*18) Ingeo6252D (manufactured by Natureworks) polylactic acid (biodegradable)

EP 3 978 079 A1

[Table 4]

| Formula B | Example | Example |
|---|---|---|
| | 18, 20 | 19 |
| Resin fiber | 1.00 | 0.50 |
| Glycerin (86%) | 5.00 | 5.00 |
| Polyoxyethylene sorbitan monostearate (20E.O.)(*19) | 0.50 | 0.50 |
| Sodium acrylate-sodium acryloyldimethyltaurate copolymer (*20) | 0.30 | 0.30 |
| Purified water | 93.20 | 93.70 |
| Total | 100.00 | 100.00 |
| Formula A | Example | Example |
| | 14, 15, 17 | 16,21 |
| Neopentyl glycol dicaprate (*21) | 17.50 | 17.50 |
| Polvoxyethylene sorbitan tristearate (20E.O.)(*22) | 1.00 | 1.00 |
| Acrylic acid-alkyl methacrylate copolymer (*23) | 0.15 | 0.15 |
| Potassium hydroxide solution (48%) | 0.10 | 0.10 |
| Resin fiber | 2.00 | 4.00 |
| Purified water | 78.90 | 76.90 |
| Phenoxyethanol | 0.35 | 0.35 |
| Total | 100.00 | 100.00 |

| Formula C | Example | |
|---|---|---|
| | 22, 23 | |
| Glycerin | 10.00 | |
| 1,3-BG | 7.50 | |
| Polyoxyethylene sorbitan tristearate (20E.O.)(*22) | 1.00 | |
| Acrylic acid-alkyl methacrylate copolymer (*23) | 0.70 | |
| Potassium hydroxide solution (48%) | 0.47 | |
| Resin fiber | 4.00 | |
| Purified water | 6.33 | |
| 95% Ethanol | 70.00 | |
| Total | 100.00 | |

*19: RHEODOL TW-S120V (Kao Corporation)
*20: SIMULGEL EG (SEPPIC)
*21: ESTEMOL N-01 (The Nisshin Oillio Group, Ltd.)
*22: RHEODOL TW-S320V (Kao Corporation)
*23: PEMULEN TR-1 (Lubrizol Advanced Materials)

Examples 14 to 23

The thin composition for forming a coating films of Table 4 were produced, and each uniformly applied to an artificial leather to form a thin film. The properties of the formed thin films were evaluated.

(Evaluation Method)

(1) Adhesivity

The thin composition for forming a coating films of formulas A, B and C were each applied to two substrates: an artificial leather and a PET film at 2 mg/cm$^2$ with a finger, and adhesivity was evaluated through bending tests on the artificial leathers and the PET films provided each with a thin film. Bending to 180 degrees was repeated ten times with the thin film on the inner side, and the state of adhesivity between the thin film and the substrate was evaluated on the basis of the following criteria.

> 4: Not changed.
> 3: The thin film is peeled or twisted at the seventh bending.
> 2: The thin film is peeled or twisted at the third bending.
> 1: The thin film is peeled or twisted at the first bending.

(2) Network formation

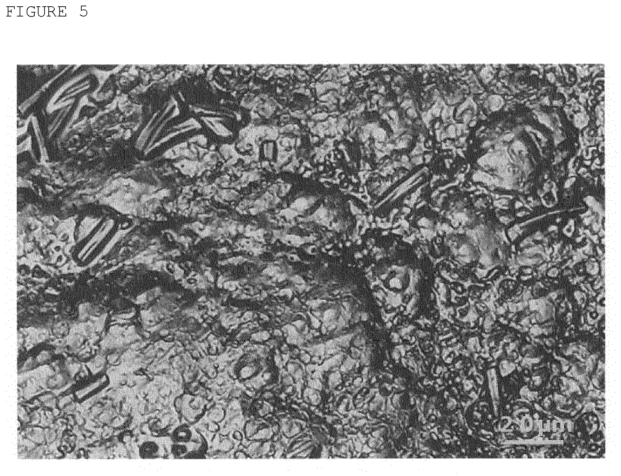

It was evaluated by SEM whether a network was formed.
In evaluation of network formation, when fibers each had two or more intersections with other fibers, and presence of gaps surrounded by the fibers was recognizable overall, it was determined that the network was formed, which was marked with "b" in the table. Figure 4 shows a SEM reference image in which the network is formed and Figure 5 shows a SEM reference image in which the network is not formed.

Reference Signs List

| | |
|---|---|
| 10 | Electrostatic spray apparatus |
| 11 | Syringe |
| 12 | High-voltage source |
| 13 | Conductive collector |
| 11a | Cylinder |
| 11b | Piston |
| 11c | Capillary |

**Claims**

1. A composition for forming a coating film,
   comprising the following components (a) and (b):

   (a) a fiber, at 0.5 mass% or more and 10 mass% or less based on the total composition for forming a coating film, having an average fiber diameter of 0.1 $\mu$m or more and 7 $\mu$m or less and an aspect ratio [(average fiber length)/(average fiber diameter)] of 8 or more and 300 or less; and
   (b) a volatile component at 15 mass% or more and 90 mass% or less based on the total composition for forming a coating film, and

   having [(average fiber diameter) $^2$/(fiber content)] ($\mu$m$^2$/mass%) being 0.005 or more and 7 or less, and a total content of the component (a) and the component (b) being 97 mass% or less.

2. The composition for forming a coating film according to claim 1, wherein the component (a) comprises a water-insoluble polymer.

3. The composition for forming a coating film according to claim 1 or 2, wherein the component (a) comprises one or more selected from the group consisting of fully saponified polyvinyl alcohols which can be subjected to insoluble treatment after film formation, partially saponified polyvinyl alcohols which can be subjected to crosslinking treatment after film formation when used in combination with a crosslinker, acrylic resins such as polymethacrylic acid resins, polyvinyl butyral resins, polyurethane resins, polylactic acid, oxazoline-modified silicones such as poly(N-propanoylethyleneimine)graft-dimethylsiloxane/y-aminopropylmethylsiloxane copolymers, polyvinyl acetal diethylaminoacetate and Zein, preferably comprises one or more selected from the group consisting of polyvinyl butyral resins, acrylic resins, polypropylene resins, polyurethane resins, polylactic acid, polybutylene succinate, polyglycolic acid, polycaprolactone and polyhydroxyalkanoic acids.

4. The composition for forming a coating film according to any one of claims 1 to 3, wherein the component (a) is polylactic acid.

5. The composition for forming a coating film according to any one of claims 1 to 4, wherein a content of the component (a) is 0.5 mass% or more and 10 mass% or less, preferably 0.7 mass% or more and 8 mass% or less, more preferably 1 mass% or more and 6 mass% or less.

6. The composition for forming a coating film according to any one of claims 1 to 5, wherein the average fiber diameter of the component (a) is 0.2 $\mu$m or more and 5 $\mu$m or less, preferably 0.3 $\mu$m or more and 4 $\mu$m or less, more preferably 0.3 $\mu$m or more and 3 $\mu$m or less.

7. The composition for forming a coating film according to any one of claims 1 to 6, wherein the average fiber length of the component (a) is 20 $\mu$m or more and 300 $\mu$m or less, preferably 25 $\mu$m or more and 250 $\mu$m or less, more preferably 30 $\mu$m or more and 200 $\mu$m or less, even more preferably 40 $\mu$m or more and 200 $\mu$m or less.

8. The composition for forming a coating film according to any one of claims 1 to 7, wherein the aspect ratio [(average fiber length)/(average fiber diameter)] of the component (a) is 8 or more and 300 or less, preferably 10 or more and 300 or less, more preferably 15 or more and 250 or less, further more preferably 20 or more and 250 or less, even more preferably 20 or more and 200 or less.

9. The composition for forming a coating film according to any one of claims 1 to 8, wherein a CV value of the fiber length of the component (a) is 40% or more and 100% or less, preferably 42% or more and 95% or less, more preferably 45% or more and 90% or less.

10. The composition for forming a coating film according to any one of claims 1 to 9, wherein the [(average fiber diameter)$^2$/ (fiber content)] ($\mu$m$^2$/mass%) in the composition is 0.02 or more and 7 or less, preferably 0.02 or more and 6 or less, more preferably 0.03 or more and 5 or less, even more preferably 0.05 or more and 4 or less.

11. The composition for forming a coating film according to any one of claims 1 to 10, wherein the component (b) comprises one or more selected from the group consisting of water, alcohol, amides, volatile silicone and volatile hydrocarbons, preferably one or more selected from water, alcohol, volatile silicone and volatile hydrocarbons.

12. The composition for forming a coating film according to any one of claims 1 to 11, wherein the component (b) comprises one or more selected from the group consisting of water, ethanol, silicone and isododecane.

13. The composition for forming a coating film according to any one of claims 1 to 12, wherein the content of the component (b) is 17 mass% or more and 87 mass% or less, preferably 17 mass% or more and 85 mass% or less, more preferably 20 mass% or more and 85 mass% or less, even more preferably 30 mass% or more and 85 mass% or less.

14. The composition for forming a coating film according to any one of claims 1 to 13, wherein the content of the component (b) is 17 mass% or more and 85 mass% or less, and the content of the component (a) is 0.7 mass% or more and 8 mass% or less, preferably 1 mass% or more and 6 mass% or less.

15. The composition for forming a coating film according to any one of claims 1 to 14, wherein the total content of the component (a) and the component (b) is preferably 16 mass% or more and 97 mass% or less, more preferably 21 mass% or more and 90 mass% or less, even more preferably 31 mass% or more and 90 mass% or less.

16. The composition for forming a coating film according to any one of claims 1 to 15, wherein the total content of the component (a) and the component (b) is preferably 21 mass% or more and 90 mass% or less.

17. The composition for forming a coating film according to any one of claims 1 to 16, wherein the mass ratio of the component (a) to the component (b) (a/b) is 0.005 or more and 0.7 or less, preferably 0.005 or more and 0.4 or less, more preferably 0.001 or more and 0.25 or less, especially preferably 0.001 or more and 0.2 or less.

18. The composition for forming a coating film according to any one of claims 1 to 17, wherein the mass ratio of the component (a) to the component (b) (a/b) is 0.01 or more and 0.25 or less.

19. The composition for forming a coating film according to any one of claims 1 to 18, further comprising one or more selected from the group consisting of (c) a nonvolatile oil agent and (e) a polyol.

20. The composition for forming a coating film according to claim 19, wherein the (c) nonvolatile oil agent comprises one or more nonvolatile oil agents selected from the group consisting of an ester oil, a hydrocarbon oil, a higher alcohol, an ether oil, a silicone oil and a fluorine oil.

21. The composition for forming a coating film according to claim 19 or 20, wherein the content of the (c) nonvolatile oil agent is 2 mass% or more and 80 mass% or less, preferably 5 mass% or more and 75 mass% or less, more preferably 8 mass% or more and 65 mass% or less.

22. The composition for forming a coating film according to any one of claims 1 to 21, whose viscosity at 20°C is 5 mPa·s or more and 50,000 mPa·s or less.

23. The composition for forming a coating film according to any one of claims 1 to 22, wherein the viscosity at 20°C is 10 mPa·s or more and 30,000 mPa·s or less.

24. A method for producing a coating film on a surface of a skin, comprising applying the composition for forming a coating film according to any one of claims 1 to 23 to the skin.

25. A short fiber for forming a thin film, wherein an average diameter of the short fiber is 0.1 $\mu$m or more and 7 $\mu$m or less, an average length of the short fiber is 20 $\mu$m or more and 300 $\mu$m or less, a CV value of the length of the short fiber is 40% or more and 100% or less, and a proportion of the number of fiber having a length of 40 $\mu$m or more in the whole short fiber is 5% or more and 100% or less.

26. The short fiber for forming a thin film according to claim 25, wherein the proportion of the number of fiber having a length of 40 $\mu$m or more in the whole fiber is 8% or more and 100% or less, preferably 15% or more and 100% or less.

27. The short fiber according to claim 25 or 26, comprising a water-insoluble polymer.

28. The short fiber according to any one of claims 25 to 27, having biodegradability.

29. The short fiber according to any one of claims 25 to 28, comprising a thermoplastic resin having an average molecular weight of $1.0 \times 10^4$ g/mol or more and $2.0 \times 10^5$ g/mol or less.

30. The short fiber according to any one of claims 25 to 29, wherein an aspect ratio of the short fiber [(average fiber length)/(average fiber diameter)] is 8 or more and 300 or less.

31. The short fiber according to any one of claims 25 to 30, wherein an aspect ratio of the short fiber [(average fiber length)/(average fiber diameter)] is 10 or more and 300 or less, preferably 15 or more and 250 or less, more preferably 20 or more and 250 or less, even more preferably 25 or more and 200 or less.

32. The short fiber according to any one of claims 25 to 31, wherein the average diameter of the short fiber is 0.2 $\mu$m or more and 5 $\mu$m or less.

33. The short fiber according to any one of claims 25 to 32, wherein the average diameter of the short fiber is 0.3 $\mu$m or more and 4 $\mu$m or less, preferably 0.3 $\mu$m or more and 3 $\mu$m or less.

34. The short fiber according to any one of claims 25 to 33, wherein an average length of the short fiber is 25 $\mu$m or more and 250 $\mu$m or less.

35. The short fiber according to any one of claims 25 to 34, wherein an average length of the short fiber is 30 $\mu$m or more and 200 $\mu$m or less, preferably 40 $\mu$m or more and 200 $\mu$m or less, even more preferably 40 $\mu$m or more and 150 $\mu$m or less.

36. The short fiber according to any one of claims 25 to 35, wherein the CV value of the length of the short fiber is 42% or more and 95% or less.

37. The short fiber according to any one of claims 25 to 36, wherein the CV value of the length of the short fiber is 45% or more and 90% or less, more preferably 45% or more and 85% or less.

38. A method for producing the short fiber according to any one of claims 25 to 37, comprising shortening nanofibers produced by an electrospinning method.

39. A coating film comprising the short fiber according to any one of claims 25 to 38.

40. The coating film according to claim 39, having a thickness of 0.3 $\mu$m or more and 30 $\mu$m or less, preferably 0.5 $\mu$m or more and 20 $\mu$m or less.

FIGURE 1

FIGURE 2

FIGURE 3

FIGURE 4

FIGURE 5

**INTERNATIONAL SEARCH REPORT**

| International application No. |
| --- |
| PCT/JP2020/021417 |

**A. CLASSIFICATION OF SUBJECT MATTER**

A61Q 1/00(2006.01)i; D01F 6/00(2006.01)i; D01F 6/38(2006.01)i; D04H 1/728(2012.01)i; D01D 5/04(2006.01)i; A61K 8/:31(2006.01)i; A61K 8/34(2006.01)i; A61K 8/81(2006.01)i; A61K 8/85(2006.01)i; A61K 8/89(2006.01)i

FI: D01F6/00 Z; A61K8/81; A61K8/34; A61K8/89; A61K8/31; A61Q1/00; D01F6/38; D01D5/04; D04H1/728; A61K8/85

According to International Patent Classification (IPC) or to both national classification and IPC

**B. FIELDS SEARCHED**

Minimum documentation searched (classification system followed by classification symbols)

A61Q1/00; D01F6/00; D01F6/38; D04H1/728; D01D5/04; A61K8/31; A61K8/34; A61K8/81; A61K8/85; A61K8/89

Documentation searched other than minimum documentation to the extent that such documents are included in the fields searched

| | |
| --- | --- |
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X<br>Y | JP 2001-064153 A (HYODO, Shoji) 13.03.2001 (2001-03-13) claim 1, paragraphs [0004], [0006], example 3 | 1-3, 5-18, 22-27, 29-37, 39-40<br>4-40 |
| Y | JP 2018-108991 A (KAO CORP.) 12.07.2018 (2018-07-12) claims 1, 4, paragraphs [0020], [0035], [0047], [0054] | 4-40 |
| A | JP 2007-051106 A (TORAY INDUSTRIES, INC.) 01.03.2007 (2007-03-01) | 1-40 |
| A | JP 2015-209393 A (KAO CORP.) 24.11.2015 (2015-11-24) | 1-40 |

☒ Further documents are listed in the continuation of Box C.     ☒ See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| 21 July 2020 (21.07.2020) | 04 August 2020 (04.08.2020) |

| Name and mailing address of the ISA/<br>Japan Patent Office<br>3-4-3, Kasumigaseki, Chiyoda-ku,<br>Tokyo 100-8915, Japan | Authorized officer<br><br><br>Telephone No. |
| --- | --- |

Form PCT/ISA/210 (second sheet) (January 2015)

**EP 3 978 079 A1**

<table>
<tr><th colspan="2" rowspan="2">INTERNATIONAL SEARCH REPORT</th><th>International application No.</th></tr>
<tr><td>PCT/JP2020/021417</td></tr>
</table>

C (Continuation).    DOCUMENTS CONSIDERED TO BE RELEVANT

| Category* | Citation of document, with indication, where appropriate, of the relevant passages | Relevant to claim No. |
|---|---|---|
| A | JP 2013-006810 A (JNC CORPORATION) 10.01.2013 (2013-01-10) | 1-40 |
| A | JP 2017-109946 A (DKS CO., LTD.) 22.06.2017 (2017-06-22) | 1-40 |

Form PCT/ISA/210 (continuation of second sheet) (January 2015)

**INTERNATIONAL SEARCH REPORT**
Information on patent family members

International application No.

PCT/JP2020/021417

| Patent Documents referred in the Report | Publication Date | Patent Family | Publication Date |
|---|---|---|---|
| JP 2001-064153 A | 13 Mar. 2001 | (Family: none) | |
| JP 2018-108991 A | 12 Jul. 2018 | US 2019/0343731 A1 claims 1, 4, paragraphs [0029], [0048], [0060], [0067] WO 2018/124227 A1 EP 3563935 A1 KR 10-2019-0078655 A CN 110121391 A | |
| JP 2007-051106 A | 01 Mar. 2007 | (Family: none) | |
| JP 2015-209393 A | 24 Nov. 2015 | (Family: none) | |
| JP 2013-006810 A | 10 Jan. 2013 | (Family: none) | |
| JP 2017-109946 A | 22 Jun. 2017 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002193746 A
- JP 2002293718 A
- JP HEI7196440 A
- JP 2005320506 A
- JP 2009114560 A
- JP 2012052271 A
- JP 2007092235 A